# EUROPEAN PATENT SPECIFICATION

(11) **EP 1 545 635 B1**
(45) Date of publication and mention of the grant of the patent: **01.08.2007**
(21) Application number: 03759495.9
(22) Date of filing: 22.09.2003
(51) Int. Cl.: A61L 12/14, A01N 25/32, A01N 37/06, A01N 47/44

(54) **COMPOSITIONS WITH ENHANCED ANTIMICROBIAL EFFICACY AGAINST ACANTHAMOEBAE**
ZUSAMMENSETZUNGEN MIT VERBESSERTER ANTIMIKROBIELLER WIRKSAMKEIT GEGEN ACANTHAMOEBAE
COMPOSITIONS A ACTION ANTI-MICROBIENNE RENFORCEE CONTRE i ACANTHAMOEBAE /i

(30) Priority: 30.09.2002 US 414956 P; 30.04.2003 US 427084
(43) Date of publication of application: 29.06.2005
(73) Proprietor: BAUSCH & LOMB INCORPORATED, Rochester, New York 14604-2701 (US)
(72) Inventor: BORAZJANI, Roya, Ft. Worth, TX 76132 (US); XIA, Erning, Penfield, NY 14526 (US); AMMON, Daniel, M., Jr., Penfield, NY 14526 (US); SALAMONE, Joseph, C., Fairport, NY 14450 (US); HU, Zhenze, Pittsford, NY 14534 (US); DOBIE, Alyce, K., Williamson, NY 14589 (US)
(74) Representative: Maiwald Patentanwalts GmbH
(86) International application number: PCT/US2003/030119
(87) International publication number: WO 2004/030710

(56) References cited:
- EP-A- 0 590 655
- WO-A-00/35499
- WO-A-97/36487
- WO-A-99/24543
- GB-A- 2 333 609
- US-B1- 6 323 165
- SILVANY R E ET AL: "THE EFFECT OF CURRENTLY AVAILABLE CONTACT LENS DISINFECTION SYSTEMS ON ACANTHAMOEBA-CASTELLANII AND ACANTHAMOEBA-POLYPHAGA" OPHTHALMOLOGY, vol. 97, no. 3, 1990, pages 286-290, XP009025458 ISSN: 0161-6420
- LARKIN D F P ET AL: "TREATMENT OF ACANTHAMOEBA KERATITIS WITH POLYHEXAMETHYLENE BIGUANIDE" OPHTHALMOLOGY, vol. 99, no. 2, 1992, pages 185-191, XP009025457 ISSN: 0161-6420

## Description

The present invention relates to the methods and/or compositions, particularly ophthalmic compositions such as eye drop and contact lens treating solutions, with a polycation material to enhance antimicrobial efficacy against protozoans such as *Acanthamoebae.* One preferred class of polycation materials is cationic cellulose derivatives. The present invention further relates to ophthalmic compositions with lower concentrations of antimicrobial agents, yet adequate preservative and/or disinfection efficacy, which may reduce irritation levels observed from higher concentrations of preservatives in ophthalmic compositions.

### BACKGROUND

The contact of eye tissue with bacteria such as *E. coli* may lead to various eye infections, such as microbial keratitis. The contact of eye tissue with bacteria may result when an ophthalmic solution contaminated with bacteria is instilled directly in the eye. Examples of such ophthalmic solutions distilled directly in the eye are eye drop solutions (for example, for treating dry eye) or contact lens drop solutions (for example, for wetting a contact lens while worn). Additionally, eye tissue may be contacted with bacteria by placing a contact lens on the eye where the contact lens is contaminated with bacteria. The risk of eye infection is increased when bacteria is adhered to a contact lens, since the bacterial may remain in contact with eye tissue for a prolonged period of time.

For this reason, ophthalmic compositions, such as eye drop and contact lens treating solutions, conventionally include an antimicrobial agent which acts as a preservative, i.e., the preservative inhibits growth of bacteria, as well as other infectious organisms, in case the solution becomes contaminated with such organisms. For contact lens treating solutions, the antimicrobial agents used to preserve the solution may also serve to disinfect contact lenses when rinsed or soaked with the solution. Alternately, ophthalmic compositions may include no preservative, but in such cases, the compositions are packaged in a special container that prevents contamination of the container contents, an example being single unit-dose packages where each dosage of solution is separately packaged.

Various antimicrobial agents are known for use as preservatives in ophthalmic compositions. Such antimicrobial agents should have a broad spectrum of antimicrobial activity and be non-irritating to the eye. However, many antimicrobial agents have a tendency to irritate eye tissue, especially at higher concentrations. Therefore, it is generally advantageous to employ as low as possible concentration of antimicrobial agent to avoid the risk of eye irritation.

In general, *Acanthamoebae* organisms are ubiquitous free-living protozoans, that exist in two distinct morphological forms: the trophozoite and the cyst. The trophozoite form is a free swimming form, which is relatively easy to kill. The organism encysts in an adverse environment, creating a thick protective coat making it very difficult to kill. The cyst form is a hibernating form of the organism. The organism reverts to the trophozoite form in a favorable environment. *Acanthamoebae* are present in soil, air, swimming pools, hot tubs, tap water, and contact-lens care products. Individuals who are susceptible to opportunistic pathogens such as *Acanthamoeba* include those who are chronically ill, immunocompromised, pregnant, diabetic, or suffer from liver disease or alcoholism. Immunocompromised individuals include patients with lymphoma, leukemia, or AIDS, and those taking immuno-suppressive medication such as organ transplant patients. *Acanthamoeba* infections include granulomatous amebic encephalitis and cutaneous lesions.

*Acanthamoeba* keratitis was first reported in 1974 and remained a rare infection until it became associated with contact lens wear. Keratitis caused by *Acanthamoeba* is the most serious complication of contact lens wear. *Acanthamoeba* Keratitis, caused by free-living *amoeba Acanthamoeba,* may lead to severe ocular infection, characterized by a painful protracted clinical course marked by frequent treatment failures. Symptoms of the infection, include marked pain and photophobia with paracentral ring-shaped stromal infiltration occurring in advanced stages of the disease.

*Acanthamoebae* use bacteria and fungi as a food source. Co-contamination of a contact lens care system with bacteria and fungi facilitates growth of the *Acanthamoebae* in the contact lens care system, and is thus implicated as a risk factor for *Acanthamoebic* keratitis. In immunocompetent individuals, *Acanthamoebae* cause a vision-threatening corneal infection known as *Acanthamoeba* keratitis. Many patients with *Acanthamoeba* keratitis are contact lens wearers. Current therapy includes the use of Brolene and neomycin, or clotrimazole.

The incidence of ulcerative keratitis among soft contact lens wearers in the United States has been found to be a function of contact lens wear mode. An incidence of infection of 4.1 per 10,000 daily wear patients per year and 20.9 per 10,000 extended wear patients per year has been found. Thus of the approximately 20 million contact lens wearers in the United States, over 12,000 infections (from all causes) occur yearly. *Acanthamoebic* keratitis has been reported in contact lens wearers regardless of lens type.

As *Acanthamoebae* are widespread in the environment, such organisms also can be found in contact lens storage cases. *Acanthamoeba* cysts and trophozoites can attach to all types of contact lenses and corresponding storage cases after a short exposure time. *Acanthamoeba* keratitis can be contracted from sources, which may include: contaminated tap water, home-prepared saline and chemical disinfection solutions, and minor corneal injury. The relative efficacy of current contact lens disinfectant solutions against *Acanthamoeba* is limited, resulting a real threat against *Acanthamoeba* keratitis.

In vitro tests have identified a number of compounds that may be useful in the treatment of *Acanthamoeba* keratitis, including propamidine isothionate, the aminoglycosides neomycin and paromomycin, and imidazole derivatives miconazole, clotrimazole, ketoconazole, and itraconazole. It has also been reported that polyhexamethylene biguanide is effective against *Acanthamoeba.* Larkin, et al., Ophthalmology 1992; 99:185-191.

Thus, development of a contact lens solution efficacious against *Acanthamoeba* is of critical importance.

The invention also relates to compositions which inhibit microbial infection. Microbes include parasites such as *Acanthamoeba castellanii, A. culbertsoni, A. hatchetti, A. polyphagia, A. rhysodes, Entamoeba histolytica, Giardia lamblia, Leishmania amazomen,* and *Trypanosoma cruzi,* and bacteria such as *Pseudomonas aeruginosa.*

In U.S. Pat. No. 5,382,599, to Rupp et al., it is disclosed that various polyvalent cation chelating agents, such as EDTA, are effective per se in inhibiting the growth of protozoans, including amoebae such as *Acanthamoebae.* An effective protozoan-growth inhibiting amount of a chelating agent is added directly to an eye care product such as a contact lens care solution.

U.S. Patent No. 6,323,165 to Heiler discloses compositions and methods for blocking proteinaceous deposits on hydrophilic contact lenses. The aforementioned compositions contain polyquaternium polymers that selectively bind to lenses and block such deposits.

U.S. Patent 6,274,133 to Hu et al. discloses compositions for treating a silicone-hydrogel contact lens while worn in the eye. The ophthalmic solutions include a cationic cellulosic polymer that binds to the lens and prevents the accumulation of lipids, proteins and other products to the lens, especially during periods of extended wear.

U.S. Patent No. 4,168,112 to Ellis discloses contact lens solutions especially adapted for rigid gas permeable (RGP) lenses, which contain cationic polymers that coat or form a hydrophilic polyelectrolytic complex on a lens surface. Ellis teaches an approach to solving the problem of protein deposits by trying to prevent proteins from adhering to a contact lens surface in the first place. Such a complex behaves as a hydrogel "cushion" thought to increase the wettability, hydrophilic character and/or comfort of the lens, while reducing a tendency for mucoproteins adherence to a lens surface.

U.S. Patent No. 4,443,429 to Smith et al. discloses the use in a contact lens disinfecting solution of a dimethyldiallylammonium chloride homopolymer commercially known as Merquat™ 100 (i.e., which has a molecular Weight of about 10,000 to about 1,000,000). Preferred disinfecting solution concentrations were recited therein as 0.0004 weight percent to about 0.02 weight percent (4 ppm to 200 ppm).

WO 02/34308 discloses inhibiting adhesion of bacteria to the surface of a biomedical device, such as a contact lens, by binding a cationic polysaccharide to the surface of the device.

WO 97/36487 discloses a contact lens cleaning solution containing Polyhexanide as an antimicrobal agent, particularly useful in killing Acanthamoeba. Silvany et al. published in their paper "Disinfecting solutions and Acanthamoeba" (Ophtalmology 1990; 97:286-290) a study, where contact lens disinfection systems were evaluated for their effectiveness in killing Acanthamoeba castellanii und Acanthamoeba polyphaga. They could show that solutions containing chlorhexidine were effective at short exposure times.

Moreover, despite of all the concerns with Amoebic keratitis, currently there are no ISO standard regulations or guidelines to require a specific efficacy (% log reduction) in marketed ophthalmic solutions.

Thus, there remains a need for additional methods and/or compositions, such as ophthalmic solutions, for inhibiting growth of protozoans, such as *acanthamoebae* in or on eye care products such as contact lenses, contact lens solutions and contact lens cases, in order to reduce the incidence of *acanthamoebic* keratitis and other ophthalmic pathologies due to the presence of protozoans.

It would be desirable to provide an ophthalmic composition with enhanced antimicrobial preservative efficacy that is safe, convenient and economical to use and non-irritating to eye tissue. The present invention is directed to overcoming the problems encountered in the art.

### SUMMARY OF THE INVENTION

The present invention further relates to methods which includes ophthalmic solutions, which may be in the form of drops and may include a cationic cellulosic polymer that exhibits prolonged duration in the eye.

The present invention relates to methods that contain a polycation material to enhance antimicrobial efficacy against protozoans such as *Acanthamoebae.* The present invention further relates to novel formulations with lower concentrations of antimicrobial agents within a wider pH range (up to 7.0), which may reduce irritation levels observed from higher concentrations of antimicrobial agents in compositions, such as eye drops or other solutions distilled into the eye.

Preferably, this invention relates to methods which include ophthalmic solutions, containing cationic cellulose materials to enhance the amoebacidal effects against *Acanthamoebae.*

The present invention also relates to *an Amoebacidal* contact lens solution, comprising polycation material in an aqueous solution.

### DETAILED DESCRIPTION OF THE INVENTION

As used herein, the term "ophthalmic composition" denotes a composition intended for application in the eye or intended for treating a medical device to be placed in contact with the eye such as a contact lens. Ophthalmic compositions specifically include compositions for direct instillation in the eye, including eye drop solutions such as for treating dry eye, and contact lens treating solutions distilled directly in the eye such as for rewetting a contact lens while worn. Ophthalmic compositions also include compositions instilled indirectly in the eye, such as contact lens treating solutions for treating the contact lens prior to the lens being inserted on the eye.

The term "preservative" or like terms denotes agents included in the ophthalmic compositions for the purpose of inhibiting the growth of microorganisms in the product, thereby helping to maintain sterility of the composition. The term "antimicrobial agent" denotes the specific active agent which provides the antimicrobial efficacy. The term "disinfecting agent" or like terms denotes an agent in an amount that will reduce the microbial bioburden on a contact lens by two log orders in four hours and more preferably by one log order in one hour. Most preferably, a disinfecting amount is an amount that will eliminate the microbial burden on a contact lens when used in regimen for the recommended soaking time (FDA Chemical Disinfection Efficacy Test-July, 1985 Contact Lens Solution Draft Guidelines).

In the case of contact lens treating solutions, the methods and/or compositions of the present invention may be applicable to the conventional contact lens categories: (1) hard lenses formed from materials prepared by polymerization of acrylic esters, such as polymethyl methacrylate (PMMA), (2) rigid gas permeable (RGP) lenses formed from silicone acrylates and fluorosilicone methacrylates, (3) soft, hydrogel lenses, and (4) non-hydrogel elastomer lenses.

As an example, soft hydrogel contact lenses are made of a hydrogel polymeric material, a hydrogel being defined as a cross-linked polymeric system containing water in an equilibrium state. In general, hydrogels exhibit excellent biocompatibility properties, i.e., the property of being biologically or biochemically compatible by not producing a toxic, injurious or immunological response in a living tissue. Representative conventional hydrogel contact lens materials are made by polymerizing a monomer mixture comprising at least one hydrophilic monomer, such as (meth)acrylic acid, 2-hydroxyethyl methacrylate (HEMA), glyceryl methacrylate, N,N-dimethacrylamide, and N-vinylpyrrolidone (NVP). In the case of silicone hydrogels, the monomer mixture from which the copolymer is prepared further includes a silicone-containing monomer, in addition to the hydrophilic monomer. Generally, the monomer mixture will include a crosslinking monomer, i.e., a monomer having at least two polymerizable radicals, such as ethylene glycol dimethacrylate, tetraethylene glycol dimethacrylate, and methacryloxyethyl vinylcarbonate. Alternately, either the silicone-containing monomer or the hydrophilic monomer may function as a crosslinking agent.

The present invention further relates to methods which include ophthalmic solutions, which may be in the form of drops and may include a cationic cellulosic polymer that exhibits prolonged duration in the eye.

The present invention may also be useful as a component of a contact lens cleaning, disinfecting or conditioning composition containing such materials. Thus, examples of material components that may be suitable and adapted for use, which are dependent upon characteristics needed for a particular application of the present invention are described below.

In protozoan cells, particularly *Acanthamoeba* cells, polyvalent cations such as calcium, magnesium, iron, manganese, and zinc serve as cofactors of enzymes required for metabolism. These polyvalent cations also affect the function and structure of the trophozoite by influencing the tonicity of the environment. Calcium and magnesium have been shown in the literature to be essential for *Acanthamoeba* encystment. See Neff et al., "Induction of Synchronous Encystment (Differentiation) in Acanthamoeba," Methods in Cell Physiology, vol. 1, ch. 4, pp. 55-83 (D. M. Prescott, ed., Academic Press, New York 1977). Calcium salts have also been shown to affect *Acanthamoeba* ameboid locomotion and attachment.

Applicants have thus found that the use of polymers that include polyvalent cation chelating moieties effectively inhibits protozoan cell functions, particularly cell growth, which require such cations. As used herein, a "polyvalent cation chelating polymer" is a polymer which includes at least one moiety that is capable of forming coordination bonds with a cation having a positive charge of at least 2. Such cations include, for example, Ca²⁺, Mg²⁺, Fe²⁺, Fe³⁺, Mn²⁺, Zn²⁺, Cu²⁺ and Ni²⁺. Polymers including combinations of two or more such moieties are also included within the scope of this term.

As used herein, a polyvalent cation chelating polymer "effectively inhibits" protozoan growth if exposure of a solution including a known initial number of protozoa to the polymer over a period of at least seven days results in a constant or reduced number of protozoa. Inhibition of protozoan growth includes in particular prevention of excystment of the protozoa.

The invention is effective in inhibiting the growth of protozoans including, but not limited to, *Acanthamoebae,* for example *A. polyphaga, A. castellani, A. lenticulata, A. hatchetti, A. astronyxis, A. culbertsoni, and A. rhysodes.* The invention is also effective in inhibiting the growth of other protozoans, such as amoebae of the genus *Naegleria.*

Many other ocular pathogens besides protozoa require one or more of the foregoing polyvalent cations for growth. See, e.g" Griffin, D., Fungal Physiology (John Wiley & Sons, Inc. 1981), p. 138 (essential mineral nutrients of fungi); Gottschalk, G., Bacterial Metabolism (Springer-Verlag New York, Inc. 1979, 1986) pp. 1-3 (minerals essential for bacterial nutrition). Thus, the inventive compositions of matter can inhibit the growth of harmful bacteria and fungi as well as protozoans. Exemplary ocular pathogens whose growth can be inhibited by recourse to the present invention include *P. aeruginosa, C albicans* and *S. marcescens.*

The compositions include a polycation material. The term "polycation" material denotes a material having multiple cationic moieties, such as quaternary ammonium radicals, in the same molecule. Many of the polycation materials, by themselves, do not have sufficient antimicrobial activity to adequately preserve an ophthalmic composition against a broad spectrum of microorganisms, but surprisingly, it has been found that the polycation material can enhance preservative efficacy against *E. coli* when used in conjunction with a conventional primary antimicrobial agent. Illustrative polycation materials include cationic polysaccharides, cationic proteins, cationic polynucleotides, cationic glycoproteins, cationic glycosaminoglycans, and ionene polymers, having multiple cationic molecules in the same molecule. It is understood that the polycation material is distinct from, and mutually exclusive of, the primary antimicrobial agent. In other words, some primary antimicrobial agents contain multiple cationic radicals; if a composition includes such a primary antimicrobial agent, then it would include an additional, separate polycation material to enhance the preservative efficacy.

In general, polyquaternium polymers suitable for use in the present invention are a well known class of polymers of which many variations are commercially available. The polyquaternium polymer, preferably includes an ophthalmologically suitable anionic organic or inorganic counterion. A preferred counterion may include, but are not limited to fluoride ions, chloride ions, bromide ions and the like.

For example, the polyquaterniums designated Polyquaternium-2 through Polyquaternium-44 (CTFA International Cosmetic Ingredient Dictionary) includes a number of materials which, based on the present teachings, are useful in the present invention. The polymerization techniques for the preparation of such materials are similarly well known to those skilled in the art and many variations of such techniques are similarly in practice in commerce.

In general, the polyquaternium polymers suitable for use in the present invention have a weight average molecular weight of about 5,000 to 5,000,000, preferably about 10,000 to 500,000, most preferably about 20,000 to 200,000. One preferred class of polycation materials is cationic polysaccharides, and especially, cationic cellulose derivatives. Specific examples include cellulosic polymers containing N,N-dimethylaminoethyl groups (either protonated or quaternized) and cellulosic polymers containing N,N-dimethylamino-2-hydroxylpropyl groups (either protonated or quaternized). Cationic cellulosic polymers are commercially available or can be prepared by methods known in the art. As an example, quaternary nitrogen-containing ethoxylated glucosides can be prepared by reacting hydroxyethyl cellulose with a trimethylammonium-substituted epoxide.

Various preferred cationic cellulosic polymers are commercially available, for example water-soluble polymers available under the CTFA (Cosmetic, Toiletry, and Fragrance Association) designation Polyquaternium-10. Such polymers are commercially available under the tradename UCARE® Polymer from Amerchol Corp., Edison, N.J., USA. These polymers contain quaternized NN-dimethylamino groups along the cellulosic polymer chain. Suitable cationic cellulosic materials have the following formula:

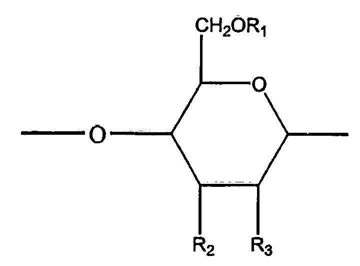

Wherein R₁ R₂ and R₃ are selected from H, derivatives of C₁-C₂₀ carboxylic acid, C₁-C₂₀ alkyl groups, C₁ to C₃ monohydric and dihydric alkanols, hydroxyethyl groups, hydroxypropyl groups, ethylene oxide groups, propylene oxide groups, phenyl groups, "Z" groups and combinations thereof. At least one of R₁ R₂ and R₃ is a Z group.

The nature of the "Z" groups is: 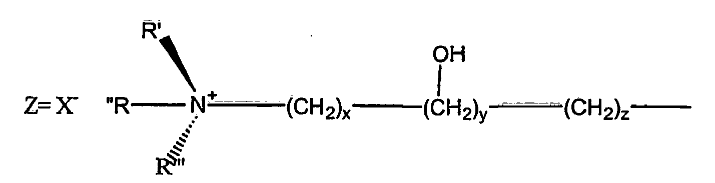 wherein: R', R" and R"' can be H, CH₃, C₂H₅, CH₂CH₂OH and CH₂CH(OH)CH₂OH

x=0-5, y=0-4, and z=0-5

X = cr, Br⁻, Γ, HSO₄-, CHSO₄⁻, H₂PO₄⁻ NO₃⁻

Various commercially available grades of the UCARE® polyquaternium-10 are summarized below:

| | **JR-125** | **JR-400** | **JR-30 M** |
|---|---|---|---|
| Brookfield Viscosity at 25°C, centipoises, 2.0% (1.7-2.2) by weight aqueous solution percent nitrogen | 110-120 cps | 400-440 cps | 12,000-13,000 cps |

In the case of eye drop solutions, the cationic polysaccharides offer the additional advantage of being effective as an active agent for treatment of dry eye. Without wishing to be bound by theory, it may be that the polymers, after binding to the mucosal tissue of the eye, in turn promote the mucin in the eye, either by supplementing the mucin and/or by helping to bind and maintain mucin on the surface of the eye. Mucins are proteins, which are heavily glycosylated with glucosamine-based moieties. Mucins have been shown to be secreted by vesicles and discharged on the surface of the conjunctival epithelium of the eye. See for example, Greiner, et al., "Mucus Secretory Vesicles in Conjunctival Epithelial Cells of Wearers of Contact Lenses," Archives of Ophthalmology, Vol. 98, pages 1843-1846 (1980). Mucins provide lubrication and additionally attract and hold moisture and sebaceous material for lubrication.

As mentioned, other polycation materials besides the cationic polysaccharides may be used in this invention. Examples include: polyquaternium-28, a polyquaternary ammonium salt based on vinylpyrrolidone and deimethylaminopropyl methacrylamide monomers (available under the tradename Gafquat HS-100, GAF Chemicals, Wayne, New Jersey, USA); hexadimethrine bromide, a polymer of N,N,N',N'-tetramethylhexamethylene-diamine and trimethylene bromide; hydroxypropyl guar triammonium chloride, a quaternary ammonium derivative of guar gum (available from Carbomer, Inc., Westborough, Massachusetts, USA); copolymers of vinyl caprolactam/PVP/dimethylaminoethyl methacrylate (such as those available under the tradename Gaffix VC-713, GAF Chemicals, Wayne, New Jersey, USA).

Other examples are polymers containing quaternary-amine-functional repeat units, defined as repeat units each comprising a quaternary-amine group, in which a positively charged nitrogen atom is covalently bonded to four radicals (no hydrogen atoms) and ionically bonded to a negatively charged counterion such as chloride.

The term "moderately charged polyquaternium polymer" as used in the present invention, may indicate that a polymer comprise not more than about 45 mole percent net quaternary-amine-functional repeat units, wherein the mole percent net quaternary-amine-functional repeat units are the mole percent of quaternary-amine-functional (positively charged) repeat units minus the mole percent of anionic (negatively charged) repeat units in the polymer.

Suitable quaternary-amine-functional repeat units also include those found in polymeric ionenes and the like formed by a polycondensation reaction; in such repeat units, the nitrogens of the quaternary-amines are integral to the polymeric backbone and are situated between alkylene, oxyalkylene, or other segments.

Quaternary-amine-functional repeat units can also be obtained as a reaction product or two or more compounds, as for example, by the use of a strong alkylating agent such as 1,4-dichloro-2-butene which, for example, can be reacted with 1,4-bis[dimethylaminol]-2-butene and triethanolamine to produce a polymeric polyquartenary ammonium compound. Quaternary-amine-functional repeat units can also be made from other polymers, such as by the reaction of a trimethyl ammonium substituted epoxide with the hydroxy group of a hydroxyethylcellulose.

The nitrogens in the quaternary-amine-funcional repeat units may be part of a saturated or unsaturated heterocyclic ring, most preferably a five- or six-membered ring. Most preferably, the polyquaternium polymer is a copolymer of a vinylimidazolium salt or a dimethyldiallyl ammonium salt. Up to 90%, preferably 40% to 90% by mole, of copolymerization-compatible comonomers not having a quaternary-amine-functionality may be copolymerized with the quaternary-amine-functional comonomers. Suitable comonomers include, but are not limited to, vinylpyrrolidone, acrylic acid, alkyl methacrylate, amides and amines such as acrylamide and N,N-dialkylaminoalkyl acrylate and methacrylate, hydroxyethylcellulose and copolymerization-compatible mixtures thereof. A preferred alkyl group has 1 to 6 carbon atoms. Most preferably, alkyl groups are methyl, ethyl, and/or butyl.

Specific polyquaternium polymers useful as a polycation material in the present invention may include, but are not limited to, copolymers in which the quaternary-amine-functional repeat units are derived from one or more of the following kinds of monomers: N,N-dimethyl-N-ethyl-aminoethyl acrylate and methacrylate, 2-methacryloxyethyltrimethylammonium, N-(3-methacrylamidopropyl)-N,N,N-trimethylammonium, 1-vinyl and 3-methyl-l-vinylimidazole, N-(3-acrylamido-3-methylbutyl)-N,N,N-trimethylammonium, N-(3-methacryloyloxy-2-hydroxypropyl)-N,N,N-trimethylammonium, diallyldimethylammonium, diallyldiethylammonium, vinylbenzyltrimethylammonium, their halides or other salt forms, and derivatives thereof, for example, involving the substitution, addition, or removal of alkyl groups, preferably having 1 to 6 carbon atoms.

A specific example of a polyquaternium copolymer is Luviquat™ FC 370 polymer (CTFA International Cosmetic Ingredient Dictionary designation polyquaternium-16 commercially available from BASF, Ludwigshafen, Germany) which is the polymerization product of a mixture of comonomers of which 70% is vinylpyrrolidone and 30% is vinylimidazolium methylchloride, commercially available as a composition with a solids content of about 40% by weight in water.

The polycation component may be employed in the compositions at about 0.001 to about 10 weight percent of the composition, preferably at about 0.005 to about 5 weight percent, with about 0.01 to about 1 weight percent being especially preferred.

As mentioned, the compositions generally include a primary antimicrobial agent. Antimicrobial agents suitable for use in the present invention include chemicals that derive their antimicrobial activity through a chemical or physiochemical interaction with the microbial organisms. These agents may be used alone or in combination.

A particularly preferred antimicrobial agent is sorbic acid (0.15%). Other known antimicrobial agents include known organic nitrogen-containing agents such as biguanides. The biguanides include the free bases or salts of alexidine, chlorhexidine, hexamethylene biguanides and their polymers, and/or combinations of the foregoing. The biguanide salts are typically gluconates, nitrates, acetates, phosphates, sulfates, halides and the like. A preferred biguanide is the hexamethylene biguanide commercially available from Zeneca, Wilmington, DE under the trademark Cosmocil™ CQ. Generally, the hexamethylene biguanide polymers, also referred to as polyhexamethylene biguanide (PHMB) and polyaminopropyl biguanide (PAPB), have molecular weights of up to about 100,000. Yet another example of a known primary antimicrobial agent is various materials available as polyquaternium-1.

The amount of the primary antimicrobial agent may vary depending on the specific agent employed. For the aforementioned organic nitrogen-containing agent, typically, such agents are present in concentrations ranging from about 0.00001 to about 0.5% weight percent, and more preferably, from about 0.00003 % to about 0.05% weight percent. For sorbic acid, higher amounts may be required, typically 0.01 to 1 weight percent, more preferably 0.1 to 0.5 weight percent. It is preferred that the antimicrobial agent is used in an amount that will at least partially reduce the microorganism population in the formulations employed. If desired, the antimicrobial agent may be employed in a disinfecting amount, which will reduce the microbial bioburden by at least two log orders in four hours and more preferably by one log order in one hour. Most preferably, a disinfecting amount is an amount which will eliminate the microbial burden on a contact lens when used in regimen for the recommended soaking time (FDA Chemical Disinfection Efficacy Test-July, 1985 Contact Lens Solution Draft Guidelines).

The aqueous solutions employed in the present invention may contain, in addition to the active ingredients described above, one or more other components that are commonly present in ophthalmic solutions, for example, tonicity adjusting agents; buffering agents; chelating agents; pH adjusting agents, viscosity modifying agents, and demulcents and the like, which aid in making ophthalmic compositions more comfortable to the user and/or more effective for their intended use.

The aqueous solutions of the present invention are typically adjusted with tonicity agents to approximate the tonicity of normal lacrimal fluids (approximately equivalent to a 0.9% solution of sodium chloride or 2.8% glycerol solution). The solutions are made substantially isotonic with physiological saline used alone or in combination with other adjusting agents. The ophthalmic compositions preferably have an osmolality of about 225 mOsm/kg to 400 mOsm/kg, more preferably 280 mOsm/kg to 320 mOsm/kg.

The compositions may include chelating or sequestering agents in order to chelate or bind metal ions, which might otherwise react with the lens and/or protein deposits and collect on the lens. Examples of such preferred materials, may include, but are not limited to ethylene-diaminetetraacetic acid (EDTA) and its salts (disodium), which are usually added in amounts ranging from about 0.01 weight percent to about 0.2 weight percent.

Compositions, such as aqueous solutions, for use in the present invention, may be formulated as lens conditioning solutions or eye-drops and sold in a wide range of small-volume containers from 1 ml to 30 ml in size. Such containers can be made from HDPE (high density polyethylene), LDPE (low density polyethylene), polypropylene, poly(ethylene terepthalate) and the like. For eye drops, flexible bottles having conventional dispensing tops are especially suitable for use with the present invention. The eye-drop formulation of the invention is used by instilling, for example, about one (1) or three (3) drops in the eye(s) as needed.

The pH of the solutions and/or compositions of the present invention may be maintained within the range of pH = 5.0 to 8.0, preferably about pH = 6.0 to 8.0, more preferably about pH = 6.5 to 7.8, most preferably pH values of greater than or equal to 7; suitable buffers may be added, such as borate, citrate, bicarbonate, tris(hydroxymethyl)aminomethane (TRIS-Base) and various mixed phosphate buffers (which may include combinations of Na₂HPO₄, NaH₂PO₄ and KH₂PO₄) and mixtures thereof. Borate buffers are preferred when the primary antimicrobial agent is PAPB. Generally, buffers will be used in amounts ranging from about 0.05 percent by weight to 2.5 percent by weight, and preferably, from 0.1 percent.by weight to 1.5 percent weight.

The compositions of this invention can be prepared by a variety of techniques conventionally used in the art. One method involves a two-phase compounding procedures. In the first phase, about 30 percent of the distilled water is used to dissolve the polymeric components (such as the cationic cellulosic polymer) with mixing for about 30 minutes at around 50 °C. The first-phase solution is then autoclaved at about 120 °C for 30 minutes. In a second phase, other components, such as alkali metal chlorides, sequestering agents, preservatives and buffering agents, are then dissolved in about 60 percent of the distilled water with agitation, followed by adding the balance of distilled water. The second-phase solution can then be sterilely added into the first-phase solution by forcing it through an 0.22 micron filter by means of pressure, followed by packaging in sterilized plastic containers.

The materials suitable for use in the present invention may also be useful as a component of a cleaning, disinfecting or conditioning solution and/or composition. Such solutions and/or compositions also may include, antimicrobial agents, surfactants, toxicity adjusting agents, buffers and the like that are known to be used components of conditioning and/or cleaning solutions for contact lenses. Examples of suitable formulations for cleaning and/or disinfecting solutions are taught in U.S. Patent 5,858,937 to Richard et al. Preferably, compositions and/or solutions of the present invention may be formulated as a "multi-purpose solution," meaning that such compositions and/or solutions may be used for cleaning, chemical disinfection, storing, and rinsing a contact lens. A multi-purpose solution preferably has a viscosity of less than 75 cps, preferably 1 to 50 cps, and most preferably 1 to 25 cps and is preferably is at least 95 percent weight by volume water in the total composition.

Surfactants, which are suitable for use in the present invention, are classified into cationic surfactants, anionic surfactants, nonionic surfactants and ampholytic surfactants depending upon their dissociation state in their aqueous solutions. Among them, various surfactants which are classified into cationic surfactants, particularly surfactants which consist of an amino acid derivative, i.e. amino acid type cationic surfactants, have conventionally been proposed as disinfectant cleaning agents or compositions for disinfection. Glycerin may also be included as a component of the present invention. Amphoteric surfactants suitable for use in a composition according to the present invention include materials of the type are offered commercially under the trade name "Miranol." Another useful class of amphoteric surfactants is exemplified by cocoamidopropyl betaine, commercially available from various sources.

Various other surfactants suitable for use in the composition can be readily ascertained, in view of the foregoing description, from McCutcheon's Detergents and Emulsifiers, North American Edition, McCutcheon Division, MC Publishing Co., Glen Rock, N.J. 07452 and the CTFA International Cosmetic Ingredient Handbook, Published by The Cosmetic, Toiletry, and Fragrance Association, Washington, D.C.

One specific class of surfactants are pluronics and reverse pluronics which are a series of ABA and BAB type block copolymers, respectively. The ABA block copolymers are composed of poly(ethylene oxide)-poly(propylene oxide)-poly(ethylene oxide) moieties, and the BAB block copolymers are composed of poly(propylene oxide)-poly(ethylene oxide)-poly(propylene oxide) blocks. The poly(ethylene oxide), PEO, blocks are hydrophilic, whereas poly(propylene oxide), PPO, blocks are hydrophobic in nature. Such materials are commercially available under the tradenames Pluoronic. The poloxamers are derived from different ratios of PEO and PPO. Another specific class of surfactants is the poloxamines, available under the tradename Tetronic, which contain blocks of PEO and PPO connected by an ethylenediamine moiety.

Optionally, one or more additional polymeric or non-polymeric demulcents may be combined with the above-named ingredients. Demulcents are known to provide wetting, moisturizing and/or lubricating effects, resulting in increased comfort. Polymeric demulcents can also act as a water-soluble viscosity builder. Included among the water-soluble viscosity builders are the non-ionic cellulosic polymers like methyl cellulose, hydroxyethyl cellulose, hydroxypropyl cellulose, and carboxymethyl cellulose, poly(N-vinylpyrrolidone), poly(vinylalcohol) and the like. Such viscosity builders or demulcents may be employed in a total amount ranging from about 0.01 to about 5.0 weight percent or less. Suitably, the viscosity of the final formulation is 10 cps to 50 cps. Comfort agents such as glycerin or propylene glycol can also be added.

### EXAMPLES

The activity of various multipurpose contact lens solutions (for cleaning, rinsing, storing and disinfecting a contact lens while the lens is not worn) and contact lens wetting drop solutions (for rewetting a contact lens while worn in the eye) were tested against the trophozoites and cysts of *Acanthamoeba polyphaga* (Ros) isolated from a case of *Acanthamoeba* keratitis. Trophozoites were grown and maintained in an axenic culture medium at 30 °C. 1 These were used to prepare cysts by transferring the trophozoites to the axenic medium supplemented with 50 mM MgC12 and incubating at 30°C with shaking for 7 days. A standard challenge test assay was used. 1 Polypropylene tubes (50 ml) were aged overnight with the test solutions; this was then discarded and 10 ml of fresh solution added. 0.1 ml of 1 x 106 /ml trophozoites or cysts as added to the solutions and at time intervals of 0, 4 or 6 hours, aliquots were removed, neutralized with 0.1% Tween 80 and the number of viable organisms determined. Control experiments used ¼ strength Ringer's solution in place of test disinfectant (¼ strength Ringer's solution: Nacl 2.25 g/l; KCl 0.105 g/l; CaCl₂.6H₂O 0.12 g/l; NaHCO₃). **References:** 1.Hughes, R. and Kilvington, S. (2001). A comparison of hydrogen peroxide contact lens disinfection systems and solutions against Acanthamoeba polyphaga. Antimicrobial Agents and Chemotherapy 45: 20382043; 2. Khunkitti, W., Lloyd, D., Furr, J.R., and Russell, A.D. (1998) Acanthamoeba castellanii: growth, encystment, excystment and biocide susceptibility. Journal of Infection 36: 43-48. The percent kill *of Acanthamoebae* after 4 hours is reported in the following tables.

The compositions in Tables 1 to 4 represent multi-purpose contact lens solutions. The compositions in Table 4 represent multi-purpose solutions that do not require a rubbing step to remove deposits from a contact lens. The compositions in Tables 5 and 6 represent contact lens rewetting drop solutions for rewetting a contact lens while worn in the eye. The compositions in Tables 7 and 8 represent multi-purpose contact lens solutions.

**Table 1**

| Ingredients | Comparative Composition | Composition 1 | Composition 2 | Composition 3 |
|---|---|---|---|---|
| | %W/W | %W/W | %W/W | %W/W |
| Boric Acid | 0.8500 | 0.8500 | 0.8500 | 0.8500 |
| Sodium Phosphate (monobasic) | 0.1500 | 0.1500 | 0.1500 | 0.1500 |
| Sodium Phosphate (Dibasic) | 0.3100 | 0.3100 | 0.3100 | 0.3100 |
| HAP (tetrasodium etidronate @ 30%) | 0.1000 | 0.1000 | 0.1000 | 0.1000 |
| Sodium Chloride | 0.1910 | 0.1910 | 0.1910 | 0.1910 |
| Cationic cellulose Polymer (Polyquaternium-10 -UCARE Polymer JR) | - | 0.0200 | 0.2000 | 1.0000 |
| PHMB HCl (Cosmocil CQ @ 20%) | 1.0 ppm | 1.0 ppm | 1.0 ppm | 1.0 ppm |
| PH | 6.96 | 6.96 | 6.95 | 7.02 |
| Osmolality | 264 | 266 | 269 | 269 |
| % Kill *Acanthamoebae* @ 4 hrs | 35% | 88% | 85% | 35% |

**Table 2**

| Ingredients | Comparative Composition | Composition 4 | Composition 5 | Composition 6 |
|---|---|---|---|---|
| | %W/W | %W/W | %W/W | %W/W |
| Boric Acid | 1.1000 | 1.1000 | 1.1000 | 1.1000 |
| Sodium Borate | 0.0900 | 0.0900 | 0.0900 | 0.0900 |
| HAP (30%) | 0.1000 | 0.1000 | 0.1000 | 0.1000 |
| Sodium Chloride | 0.3500 | 0.3500 | 0.3500 | 0.3500 |
| Polymer JR | | 0.0200 | 0.2000 | 1.0000 |
| PHMB HCl (20%) | 1.0 ppm | 1.0 ppm | 1.0 ppm | 1.0 ppm |
| PH | 7.17 | 7.15 | 7.15 | 7.15 |
| Osmo | 287 | 294 | 300 | 269 |
| % Kill *Acanthamoebae* @ 4 hrs | 15% | 75% | 75% | 32% |

**Table 3**

| Ingredients | Comparative Composition | Composition 7 | Composition 8 | Composition 9 |
|---|---|---|---|---|
| | %W/W | %W/W | %W/W | %W/W |
| Sodium Phosphate (monobasic) | 0.09625 | 0.09625 | 0.09625 | 0.09625 |
| Sodium Phosphate (Dibasic) | 0.1140 | 0.1140 | 0.1140 | 0.1140 |
| HAP (30%) | 0.1000 | 0.1000 | 0.1000 | 0.1000 |
| Sodium Chloride | 0.8010 | 0.8010 | 0.8010 | 0.8010 |
| Polymer JR | - | 0.0200 | 0.2000 | 1.0000 |
| PHMB HCl (20%) | 1.0 ppm | 1.0 ppm | 1.0 ppm | 1.0 ppm |
| PH | 6.94 | 6.93 | 6.92 | 6.94 |
| Osmo 286 | | 290 | 294 | >294 |
| % Kill *Acanthamoebae* @ 4 hrs | 10% | 11% | 20% | 7% |

**Table 4**

| Ingredients | Comp 10 | Comp 11 | Comp 12 | Comp 13 | Comp 14 |
|---|---|---|---|---|---|
| | %W/W | %W/W | %W/W | %W/W | %W/W |
| Boric Acid | 0.8500 | 0.8500 | 0.8500 | 0.8500 | 0.8500 |
| Sodium Phosphate (monobasic) | 0.1500 | 0.1500 | 0.1500 | 0.1500 | 0.1500 |
| Sodium Phosphate (Dibasic) | 0.3100 | 0.3100 | 0.3100 | 0.3100 | 0.3100 |
| HAP (30%) | 0.1000 | 0.1000 | 0.1000 | 0.1000 | 0.1000 |
| Sodium Chloride | 0.2600 | 0.1917 | 0.0880 | 0.2600 | 0.1917 |
| Polymer JR | 0.0200 | 0.0200 | 0.0200 | 0.0200 | 0.0200 |
| PHMB HC l (20%) | 1.29 ppm | 1.29 ppm | 1.29 ppm | 1.29 ppm | 1.29 ppm |
| PoloxamerF127 (Pluronic F127) | 2.0000 | 2.0000 | 2.0000 | 3.0000 | - |
| Poloxamine 1107 (Tetronic 1107) | 1.0000 | 1.0000 | 1.0000 | - | 1.0000 |
| EDTA | - | - | - | - | - |
| Sodium Borate | - | - | - | - | - |
| PH | 7.00 | 7.00 | 7.02 | 6.93 | 6.92 |
| Osmo | 300 | 282 | 258 | 290 | 294 |
| ISO Biocidal @ 4 hrs | 2.9 | 3.8 | 4.0 | 2.4 | |
| % Kill *Acanthamoebae* @ 4 hrs | 60% | 92% | 100% | 100% | 93% |

**Table 5**

| Ingredients | Composition 15 | Comparative Composition |
|---|---|---|
| | %W/W | %W/W |
| Tris-Base | 0.1210 | 0.1210 |
| Sodium Borate | 0.2198 | 0.2198 |
| Glycerin | 1.0000 | 1.0000 |
| Sodium Chloride | 0.2660 | 0.2660 |
| Tetronic 1107 | 1.0000 | 1.0000 |
| Pluonic F 127 | 2.0000 | 2.0000 |
| Polymer JR | 0.0200 | |
| Sorbic Acid | 0.2000 | 0.2000 |
| EDTA | 0.2000 | 0.2000 |
| PH | 6.79 | 7.09 |
| Osmo. | 297 | 311 |
| % Kill *Acanthamoebae* @ 4 hrs | 75% | 7% |
| PE Result | Pass | Pass |

**Table 6**

| Ingredients | Compar ative Comp | Comp 16 | Comp 17 | Comp 18 | Comp 19 | Comp 20 | Comp 21 | Comp 22 | Comp 23 |
|---|---|---|---|---|---|---|---|---|---|
| | %W/W | O/OW/W | %W/W | %W/W | %W/W | %W/W | %W/W | %W/W | %W/W |
| Tris-Base | 0.1210 | 0.1210 | 0.1210 | 0.1210 | 0.1210 | 0.1210 | 0.1210 | 0.1210 | 0.1210 |
| Sodium Borate | 0.2198 | 0.2198 | 0.2198 | 0.2198 | 0.2198 | 0.2198 | 0.2198 | 0.2198 | 0.2198 |
| Glycerin | 1.0000 | 1.0000 | 1.0000 | 1.0000 | 1.0000 | 1.0000 | 1.0000 | 1.0000 | 1.0000 |
| Sodium Chloride | 0.2660 | 0.2660 | 0.2660 | 0.2660 | 0.2660 | 0.2660 | 0.2660 | 0.2660 | 0.2660 |
| Tetronic 1107 | 1.0000 | 1.0000 | 1.0000 | 1.0000 | 1.0000 | 1.0000 | 1.0000 | 1.0000 | 1.0000 |
| Pluonic F127 | 2.0000 | 2.0000 | 2.0000 | 2.0000 | 2.0000 | 2.0000 | 2.0000 | 2.0000 | 2.0000 |
| Polymer JR | -- | 0.0010 | 0.0050 | 0.0100 | 0.0200 | 0.0400 | 0.1000 | 0.4000 | 0.8000 |
| Sorbic Acid | 0.2000 | 0.2000 | 0.2000 | 0.2000 | 0.2000 | 0.2000 | 0.2000 | 0.2000 | 0.2000 |
| EDTA | 0.2000 | 0.2000 | 0.2000 | 0.2000 | 0.2000 | 0.2000 | 0.2000 | 0.2000 | 0.2000 |
| PH | 6.79 | 6.79 | 6.79 | 6.79 | 6.79 | 6.79 | 6.79 | 6.79 | 6.79 |
| Osmo. | 297 | 297 | 297 | 297 | 297 | 297 | 297 | 297 | 297 |
| % Kill *Acanthamoebae* @4hrs | 5.65 | 47.28 | 64.33 | 72.59 | 75.73 | 79.29 | 86.51 | 88.28 | 91.09 |

**Table 7**

| Ingredients | Compar ative Comp | Comp 24 | Comp 25 | Comp 26 | Comp 27 | Comp 28 | Comp 29 | Comp 30 | Comp 31 |
|---|---|---|---|---|---|---|---|---|---|
| | %W/W | %W/W | %W/W | %W/W | %W/W | %W/W | %W/W | %W/W | %W/W |
| Boric Acid | 0.8500 | 0.8500 | 0.8500 | 0.8500 | 0.8500 | 0.8500 | 0.8500 | 0.8500 | 0.8500 |
| Sodium Phosphate (monobasic) | 0.1500 | 0.1500 | 0.1500 | 0.1500 | 0.1500 | 0.1500 | 0.1500 | 0.1500 | 0.1500 |
| Sodium Phosphate (Dibasic)) | 0.3100 | 0.3100 | 0.31.00 | 0.3100 | 0.3100 | 0.3100 | 0.3100 | 0.3100 | 0.3100 |
| HAP (30%) | 0.1000 | 0.1000 | 0.1000 | 0.1000 | 0.1000 | 0.1000 | 0.1000 | 0.1000 | 0.1000 |
| Sodium Chloride | 0.1917 | 0.1917 | 0.1917 | 0.1917 | 0.1917 | 0.1917 | 0.1917 | 0.1917 | 0.1917 |
| Polymer JR | | 0.0010 | 0.0050 | 0.0100 | 0.0200 | 0.0400 | 0.1000 | 0.4000 | 0.8000 |
| PHMB HCl (20%) | 0.00779 | 0.00779 | 0.00779 | 0.00779 | 0.00779 | 0.00779 | 0.00779 | 0.00779 | 0.00779 |
| Pluronic F127 | 2.0000 | 2.0000 | 2.0000 | 2.0000 | 2.0000 | 2.0000 | 2.0000 | 2.0000 | 2.0000 |
| Tetronic 1107 | 1.0000 | 1.0000 | 1.0000 | 1.0000 | 1.0000 | 1.0000 | 1.0000 | 1.0000 | 1.0000 |
| PH | 7.00 | 7.00 | 7.00 | 7.00 | 7.00 | 7.00 | 7.00 | 7.00 | 7.00 |
| Osmo | 282 | 282 | 282 | 282 | 282 | 282 | 282 | 282 | 282 |
| % Kill *Acanthamoebae* @ 4 hrs | 9.43 | 51.24 | 82.00 | 81.14 | 87.20 | 81.33 | 94.31 | 90.90 | 65.62 |

**Table 8**

| Ingredients | Comparative Comp | Comp 32 | Comp 33 | Comp 34 | Comparative Comp | Comp 35 | Comp 36 |
|---|---|---|---|---|---|---|---|
| | %W/W | %W/W | %W/w, | %W/W | %W/W | %W/W | %W/W |
| Boric Acid | 0.8500 | 0.8500 | 0.8500 | 0.8500 | 0.8500 | 0.8500 | 0.8500 |
| Sodium Borate | 0.0900 | 0.0900 | 0.0900 | 0.0900 | 0.0900 | 0.0900 | 0.0900 |
| Sodium Chloride | 0.4500 | 0.4500 | 0.4500 | 0.4500 | 0.4500 | 0.4500 | 0.4500 |
| HAP (30%) | 0.1000 | 0.1000 | 0.1000 | 0.1000 | 0.1000 | 0.1000 | 0.1000 |
| Polycation/Other Component (0.02% when present) | - | Gafquat HS-100 | Hexadimethrine Bromide | Hydroxypropyl GuarTriAmmonium Chloride | Gluquat 125 | GAFFIX VC-713 | Polymer JR |
| PHMB HC1 (20%) | 0.00779 | 0.00779 | 0.00779 | 0.00779 | 0.00779 | 0.00779 | 0;00779 |
| % Kill *Acanthamoebae* | 11.43 | 94.17 | 99.50 | 69.05 | 4.95 | 64.48 | 74.67 |

Although preferred embodiments have been depicted and described in detail herein, it will be apparent to those skilled in the relevant art that various modifications, additions, substitutions, and the like can be made without departing from the spirit of the invention and these are therefore considered to be within the scope of the invention as defined in the claims which follow.

## Claims

1. A method for enhancing antimicrobial efficacy of a contact lens against *Acanthamoebae,* comprising contacting said contact lens with a solution comprising a primary antimicrobial agent and a cationic cellulose polymer, the solution having an osmolality of 280 mOsm/kg to 320 mOsm/kg.

2. The method according to claim 1, wherein the composition is an aqueous ophthalmic solution.

3. The method according to claim 2, wherein the composition further comprises at least one component selected from the group consisting of tonicity adjusting agents, buffering agents, pH adjusting agents, viscosity modifying agents, and therapeutic agents.

4. The method according to any one of claims 1 to 3, wherein the primary antimicrobial agent includes sorbic acid.

5. The method according to any one of claims 1 to 3, wherein the primary antimicrobial agent includes a biguanide.

6. The method according to claim 3, wherein the solution is in the form of a multipurpose contact lens solution for rinsing, storing, cleaning and disinfecting the contact lens, wherein the contact lens rinsed with said solution is suitable for placement in the eye without irritation to eye tissue.

7. The method according to any one of the previous claims, wherein the growth of *Acanthamoebae* is inhibited.

## Patentansprüche

1. Ein Verfahren zur Verbesserung der antimikrobiellen Wirksamkeit einer Kontaktlinse gegen *Acanthamoebae,* umfassend das in Kontaktbringen besagter Kontaktlinse mit einer Lösung umfassend einen primären antimikrobiellen Wirkstoff und ein kationisches Zellulosepolymer, wobei die Lösung eine Osmolalität von 280 mOsm/kg bis 320 mOsm/kg aufweist.

2. Das Verfahren gemäß Anspruch 1, wobei die Zusammensetzung eine wässrige ophthalmische Lösung ist.

3. Das Verfahren gemäß Anspruch 2, wobei die Zusammensetzung ferner zumindest einen Bestandteil enthält, der ausgewählt ist aus der Gruppe bestehend aus tonizitätsregulierenden Substanzen, Puffersubstanzen, pH-regulierenden Substanzen, viskositätsändernden Substanzen und therapeutischen Wirkstoffen.

4. Das Verfahren gemäß einem der Ansprüche 1 bis 3, wobei der primäre antimikrobielle Wirkstoff Sorbinsäure enthält.

5. Das Verfahren gemäß einem der Ansprüche 1 bis 3, wobei der primäre antimikrobielle Wirkstoff Biguanid enthält.

6. Das Verfahren gemäß Anspruch 3, wobei die Lösung in Form einer Mehrzweck-Kontaktlinsenlösung zum Spülen, Lagern, Reinigen und Desinfizieren der Kontaktlinse vorliegt, wobei die mit besagter Lösung gespülte Kontaktlinse zum Einsetzen in das Auge ohne Reizung des Augengewebes geeignet ist.

7. Das Verfahren gemäß einem der vorherigen Ansprüche, wobei das Wachstum von *Acanthamoebae* gehemmt wird.

## Revendications

1. Procédé permettant d'améliorer l'efficacité antimicrobienne d'une lentille de contact contre *Acanthamoebae,* comprenant la mise en contact de ladite lentille de contact avec une solution contenant un agent antimicrobien primaire et un polymère de cellulose cationique, la solution présentant une osmolalité de 280 mOsm/kg à 320 mOsm/kg.

2. Procédé selon la revendication 1, dans lequel la composition est une solution ophtalmique aqueuse.

3. Procédé selon la revendication 2, dans lequel la composition contient en outre au moins un composant sélectionné dans le groupe constitué d'agents d'ajustement de la tonicité, d'agents de tamponnement, d'agents d'ajustement du pH, d'agents modifiant la viscosité et d'agents thérapeutiques.

4. Procédé selon l'une des revendications 1 à 3, dans lequel l'agent antimicrobien primaire contient de l'acide sorbique.

5. Procédé selon l'une des revendications 1 à 3, dans lequel l'agent antimicrobien primaire contient une biguanide.

6. Procédé selon la revendication 3, dans lequel la solution se présente sous la forme d'une solution de lentille de contact multi-usage pour le rinçage, le stockage, le nettoyage et la désinfection de la lentille de contact, la lentille de contact rincée avec ladite solution étant appropriée pour un positionnement sur l'oeil sans irritation des tissus oculaires.

7. Procédé selon l'une des revendications précédentes, dans lequel la croissance de *Acanthamoebae* est inhibée.
